(19) **Europäisches Patentamt**
**European Patent Office**
**Office européen des brevets**

(11) **EP 4 674 956 A1**

(12) **EUROPEAN PATENT APPLICATION**
published in accordance with Art. 153(4) EPC

(43) Date of publication:
**07.01.2026 Bulletin 2026/02**

(21) Application number: **24764032.9**

(22) Date of filing: **01.03.2024**

(51) International Patent Classification (IPC):
***C12N 9/26*** (2006.01)   ***A23L 33/21*** (2016.01)
***C12N 9/28*** (2006.01)

(52) Cooperative Patent Classification (CPC):
**A23L 33/21; C12N 9/2408; C12N 9/2414**

(86) International application number:
**PCT/JP2024/007761**

(87) International publication number:
**WO 2024/181555 (06.09.2024 Gazette 2024/36)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **02.03.2023 JP 2023032204**

(71) Applicant: **Amano Enzyme Inc.**
**Nagoya-shi**
**Aichi 460-8630 (JP)**

(72) Inventor: **SAKAI, Kiyota**
**Kakamigahara-shi, Gifu 509-0109 (JP)**

(74) Representative: **Mullholland, Lewis Paul**
**WP Thompson**
**44 Southampton Buildings**
**London WC2A 1AP (GB)**

(54) **ENZYME PREPARATION FOR PRODUCING BETA-GLUCAN-CONTAINING PLANT-BASED
LIQUID COMPOSITION**

(57)   [Problems] The main object of the present invention is to provide a novel technique for producing a β-glucan-containing plant-based liquid composition simply and at low cost.

[Solution] This technique provides an enzyme preparation for producing β-glucan-containing plant-based liquid composition, containing α-amylase and β-glucanase. This technique also provides an agent for increasing the β-glucan content of a plant-based liquid composition, containing α-amylase and β-glucanase. This technique further provides a method for producing a β-glucan-containing plant-based liquid composition, which includes an enzyme treating step of allowing α-amylase and β-glucanase to treat on a starch-containing plant-based material.

**EP 4 674 956 A1**

**Description**

Technical Field

The present technique relates to an enzyme preparation for producing β-glucan-containing plant-based liquid composition. More specifically, the present technique relates to an enzyme preparation for producing β-glucan-containing plant-based liquid composition that can increase the β-glucan content, an agent for increasing the β-glucan content in a plant-based liquid composition, and a method for producing a β-glucan-containing plant-based liquid composition.

Background Art

Drinks rich in nutrients such as protein (e.g., milk) have traditionally been popular because they allow people to easily ingest nutrients. However, due to the recent increase in vegetarians, allergy issues, religious reasons, and other factors, drinks made from raw materials such as oats, which are rich in plant-based protein, have become widely popular, as an alternative to animal milks such as cow's milk.

Against this background, there has been a growing need in recent years for liquid compositions containing processed plant-based proteins to provide physiological effects that have not previously been achieved with animal-based foods such as milk. For example, oat milk has seen a growth in its market in recent years due to its high content of β-glucan, known as a type of water-soluble dietary fiber, and the like. β-glucan has been recognized to have effects such as lowering cholesterol levels and suppressing blood sugar levels.

However, because plant-based materials such as oats contain high concentrations of starch, there is a problem that when treated with a liquefying enzyme (such as α-amylase) during the production step, the β-glucan is decomposed due to the contaminating β-glucanase activity contained in the liquefying enzyme preparation. To solve this problem, for example, Patent Document 1 discloses a method for preparing an oat suspension, which includes a step of treating the oat suspension with an enzyme preparation having α-amylase activity with no β-glucan-digesting enzyme.

Citation List

Patent Literature

Patent Literature 1: Japanese Translation of PCT Publication No. 09-505204

Summary of Invention

Technical Problem

As mentioned above, a technique for suppressing the decomposition of β-glucan has been developed that uses an enzyme preparation that does not contain a β-glucan digesting enzyme. However, there is a problem that it is not practical because it needs effort and cost to completely remove the β-glucanase contained in the liquefying enzyme.

Therefore, the main object of the present invention is to provide a novel technique for producing a β-glucan-containing plant-based liquid composition simply and at low cost.

Solution to Problem

As a result of extensive research, the inventors of the present invention have found that the β-glucan content in a plant-based liquid composition can be maintained at a certain level or higher even when a liquefying enzyme containing β-glucanase is used. Based on this finding, the present invention was completed through further research.

That is, the present technique first provides an enzyme preparation for producing β-glucan-containing plant-based liquid composition, which contains α-amylase and β-glucanase.

The content of the β-glucanase in the enzyme preparation for producing β-glucan-containing plant-based liquid composition according to the present technique can be 0.001 U or more and 300 U or less per 1 g of β-glucan in the raw materials of the β-glucan-containing plant-based liquid composition.

The enzyme preparation for producing β-glucan-containing plant-based liquid composition according to the present technique can be used to produce plant-based milk as a β-glucan-containing plant-based liquid composition.

Next, the present technique provides an agent for increasing the β-glucan content of a plant-based liquid composition, which contains α-amylase and β-glucanase.

The present technique further provides a method for producing a β-glucan-containing plant-based liquid composition, comprising an enzyme treating step of allowing α-amylase and β-glucanase to treat on a starch-containing plant-based material.

In the enzyme treating step of the production method according to the present technique, the β-glucanase can be allowed to treat at 0.001 U or more and 300 U or less per 1 g of β-glucan in the starch-containing plant-based material.

Advantageous Effects of Invention

According to this technique, a plant-based liquid composition with a high β-glucan content can be produced without completely removing the β-glucanase contained in the liquefying enzyme.

Description of Embodiments

Preferred embodiments for carrying out the present technique will be described below. Note that the embodiments described below are examples of typical embodiments of the present technique, and the scope of the present technique should not be construed as being narrow by them.

1.Enzyme preparation for producing β-glucan-containing plant-based liquid composition, and agent for increasing the β-glucan content of plant-based liquid composition

The enzyme preparation for producing β-glucan-containing plant-based liquid composition and the agent for increasing the β-glucan content of a plant-based liquid composition according to the present technique contain α-amylase and β-glucanase. As demonstrated in the examples below, the present technique has discovered that the β-glucan content in a plant-based liquid composition can be maintained at a certain level or higher even when a liquefying enzyme containing β-glucanase is used. Furthermore, by adjusting the amount of β-glucanase in the enzyme preparation to a certain range, the β-glucan content in the produced plant-based liquid composition was successfully increased. Below, the components that can be used in the enzyme preparation for producing β-glucan-containing plant-based liquid composition and the agent for increasing the β-glucan content of a plant-based liquid composition according to the present technique will be described in detail.

(1) α-amylase

α-amylase is an enzyme that treats on starch and mainly hydrolyzes α-1,4-glucosidic bonds. α-amylase that can be used in the present technique may be an enzyme that also has other actions, as long as it has α-amylase activity and does not impair the action and effect of the present technique.

The origin of α-amylase that can be used in the present technique is not particularly limited as long as it does not impair the action and effect of the present technique. Examples thereof include filamentous fungi such as Aspergillus aureus, Aspergillus foetidus, Aspergillus niger, and Aspergillus oryzae; actinomycetes such as Saccharomonospora viridis, Streptomyces avermitilis, Streptomyces griseus, Streptomyces thermoviolaceus, Streptomyces violaceoruber, and Thermomonospora viridis; and bacteria such as Alcaligenes latus, genus Arthrobacter, Bacillus amyloliquefaciens, Bacillus circulans, Bacillus licheniformis, Bacillus stearothermophilus, Bacillus subtilis, Cellulosimicrobium cellulans, Microbacterium imperiale, Paenibacillus alginolyticus, and Sulfolobus solfataricus. Preferably, the α-amylase is derived from the genus Bacillus or Aspergillus, and more preferably, the α-amylase is derived from Bacillus amyloliquefaciens or Bacillus licheniformis.

In this technique, not only natural (wild-type) α-amylase but also recombinant α-amylase can be used. Commercially available α-amylase or α-amylase preparations can also be used. An example of a commercially available α-amylase or α-amylase preparation is the α-amylase derived from Bacillus amyloliquefaciens manufactured by Amano Enzyme Inc.

The α-amylase used in the present technique can be prepared from a culture solution of a microorganism from which the above-mentioned α-amylase is derived. Specific preparation methods include recovering the α-amylase from the culture solution or bacterial bodies of the above-mentioned microorganism. For example, when an α-amylase-secreting microorganism is used, the bacterial bodies can be recovered from the culture solution in advance by filtration, centrifugation, or the like, as necessary, and the enzyme can then be separated and/or purified. In contrast, when an α-amylase-nonsecreting microorganism is used, the bacterial bodies can be recovered from the culture solution in advance, as necessary, and then the bacterial bodies may be disrupted by pressure treatment, ultrasonic treatment, or the like to extract the enzyme, after which the enzyme can be separated and/or purified. The enzyme can be separated and/or purified by any known protein separation and/or purification method, without any particular limitation. Examples thereof include centrifugation, UF concentration, salting out, and various chromatography methods using ion exchange resins, etc. The separated and/or purified enzyme can be powdered by drying methods such as freeze-drying and vacuum drying, or can also be powdered using appropriate excipients and/or drying aids in the drying methods. The separated and/or purified enzyme can also be formulated as a liquid by adding an appropriate additive and sterilizing by filtration.

The content of α-amylase in the enzyme preparation for producing β-glucan-containing plant-based liquid composition and the agent for increasing the β-glucan content of a plant-based liquid composition according to the present technique is not particularly limited as long as it does not impair the action and effect of the present technique. The lower limit of the content of α-amylase can be set to, for example, 0.0005 U or more, preferably 0.001 U or more, 0.005 U or more, more preferably 0.01 U or more, 0.03 U or more, 0.05 U or more, and even more preferably 0.1 U or more, 0.5 U or more, 1 U or more, 5 U or more, or 10 U or more per 1 g of the plant-based material in the β-glucan-containing plant-based liquid composition. Furthermore, the lower limit of the content of α-amylase can be set to, for example, 0.001 U or more, preferably 0.005 U or more, more preferably 0.01 U or more, 0.03 U or more, 0.05 U or more, and even more preferably 0.1 U or more, 0.5 U or more, 1 U or more, 5 U or more, or 10 U or more per 1 g of the carbohydrate (preferably, starch) contained in the plant-based material of the β-glucan-containing plant-based liquid composition.

On the other hand, the upper limit of the content of α-amylase can be set to, for example, 500,000 U or less, 100,000 U or less, 50,000 U or less, 10,000 U or less, 5,000 U or less, preferably 4,000 U or less, 2,000 U or less, 1,000 U or less, more preferably 800 U or less, 600 U or less, 400 U or less, 200 U or less, 100 U or less, 80 U or less, per 1 g of the plant-based material in the β-glucan-containing plant-based liquid composition. Furthermore, the upper limit of the content of α-amylase can be set to, for example, 500,000 U or less, 100,000 U or less, 50,000 U or less, 10,000 U or less, 5,000 U or less, preferably 4,000 U or less, 2,000 U or less, 1,000 U or less, more preferably 800 U or less, 600 U or less, 400 U or less, 200 U or less, 100 U or less, per 1 g of the carbohydrate (preferably, starch) contained in the plant-based material of the β-glucan-containing plant-based liquid composition.

In this technique, one unit (1 U) of α-amylase activity is defined as the amount of enzyme that reduces the color of potato starch caused by iodine by 10% in one minute when treated with 1% potato starch as a substrate at pH 5.0 and 37° C. In this technique, the α-amylase activity is a value measured by the α-amylase activity measurement method described in the Examples below.

(2) β-glucanase

β-glucanase is an enzyme that hydrolyzes the glucosidic bond of β-glucan. The β-glucanase that can be used in the present technique may be an enzyme that also has other actions, as long as it has β-glucanase activity.

The origin of the β-glucanase that can be used in the present technique is not particularly limited as long as it does not impair the action and effect of the present technique. β-glucanase derived from a microorganism that produces a liquefying enzyme or a raw material used in the production step of the liquefying enzyme can be used. Preferably, β-glucanase derived from a microorganism that produces a liquefying enzyme can be used. Examples thereof specifically include filamentous fungi such as Aspergillus aureus, Aspergillus foetidus, Aspergillus niger, and Aspergillus oryzae; actinomycetes such as Saccharomonospora viridis, Streptomyces avermitilis, Streptomyces griseus, Streptomyces thermoviolaceus, Streptomyces violaceoruber, and Thermomonospora viridis; and bacteria such as Alcaligenes latus, genus Arthrobacter, Bacillus amyloliquefaciens, Bacillus circulans, Bacillus licheniformis, Bacillus stearothermophilus, Bacillus subtilis, Cellulosimicrobium cellulans, Microbacterium imperiale, Paenibacillus alginolyticus, and Sulfolobus solfataricus. The β-glucanase is preferably derived from the genus Bacillus or Aspergillus, and more preferably derived from Bacillus amyloliquefaciens or Bacillus licheniformis.

In this technique, not only natural (wild-type) β-glucanase but also recombinant β-glucanase can be used. Commercially available β-glucanase or β-glucanase preparations can also be used.

The β-glucanase used in the present technique can be prepared from a culture solution of a microorganism from which the above-mentioned β-glucanase is derived. Specific preparation methods include methods of recovering the β-glucanase from the culture solution or bacterial bodies of the above-mentioned microorganism. For example, when a β-glucanase-secreting microorganism is used, the bacterial bodies can be recovered from the culture solution in advance by filtration, centrifugation, or the like, as necessary, and the enzyme can then be separated and/or purified. When a β-glucanase-nonsecreting microorganism is used, the bacterial bodies can be recovered from the culture solution in advance, as necessary, and then the bacterial bodies may be disrupted by pressure treatment, ultrasonic treatment, or the like to extract the enzyme, after which the enzyme can then be separated and/or purified. The enzyme can be separated and/or purified by any known protein separation and/or purification method, without any particular limitation. Examples thereof include centrifugation, UF concentration, salting out, and various chromatography methods using ion exchange resins, etc. The separated and/or purified enzyme can be powdered by drying methods such as freeze drying and vacuum drying, and can also be powdered using appropriate excipients and/or drying aids in the drying methods. The separated and/or purified enzyme can also be formulated as a liquid by adding an appropriate additive and sterilizing by filtration.

The content of β-glucanase in the enzyme preparation for producing β-glucan-containing plant-based liquid composition and the agent for increasing the β-glucan content of a plant-based liquid composition according to the present technique is not particularly limited as long as it does not impair the action and effect of the present technique. The lower limit of the content of β-glucanase can be set to, for example, 0.00001 U or more, preferably 0.00005 U or more, or 0.0001 U or more per 1 g of the plant-based material in the β-glucan-containing plant-based liquid composition, and from the viewpoint of reducing the cost and effort required to reduce the β-glucanase activity contained in the liquefying enzyme preparation, it can be set to more preferably 0.0002 U or more, 0.0003 U or more, even more preferably 0.0005 U or more, 0.001 U or more, 0.005 U or more, 0.01 U or more, or 0.1 U or more. Furthermore, the lower limit of the content of β-glucanase can be set to, for example, 0.001 U or more, preferably 0.005 U or more, per 1 g of β-glucan contained in the plant-based material of the β-glucan-containing plant-based liquid composition, and from the viewpoint of reducing the cost and effort required to reduce the β-glucanase activity contained in the liquefying enzyme preparation, it can be set to more preferably 0.008 U or more, 0.01 U or more, 0.015 U or more, even more preferably 0.03 U or more, 0.05 U or more, 0.1 U or more, 0.5 U or more, 1 U or more, 5 U or more.

On the other hand, the upper limit of the content of β-glucanase can be set to, for example, 5 U or less, 4 U or less, 3 U or less, or 2 U or less per 1 g of the plant-based material in the β-glucan-containing plant-based liquid composition, and from the viewpoint of preventing degradation of β-glucan, it can be set to preferably 1.5 U or less, 1 U or less, 0.5 U or less, or 0.1 U or less, more preferably 0.08 U or less, 0.06 U or less, 0.04 U or less, 0.02 U or less, or 0.01 U or less. Furthermore, the upper limit of the content of β-glucanase can be set to, for example, 300 U or less, or 200 U or less per 1 g of β-glucan contained in the plant-based material in the β-glucan-containing plant-based liquid composition, and from the viewpoint of preventing degradation of β-glucan, it can be set to preferably 100 U or less, 80 U or less, more preferably 60 U or less, 40 U or less, 20 U or less, 15 U or less, 10 U or less, 5 U or less, 2 U or less, or 1 U or less.

In the present technique, one unit of β-glucanase activity is defined as the amount of enzyme that produces 1 μmol of glucose in 17 hours when treated with a 0.55% carboxymethylcellulose sodium solution as a substrate at pH 7.0 and 52°C. In the present technique, the β-glucanase activity is a value measured by the β-glucanase activity measurement method described in the Examples below.

The blending ratio of α-amylase to β-glucanase in the enzyme preparation for producing β-glucan-containing plant-based liquid composition and the agent for increasing the β-glucan content of a plant-based liquid composition according to the present technique is not particularly limited, as long as it does not impair the action and effect of the present technique. The blending ratio of α-amylase to β-glucanase in the enzyme preparation for producing β-glucan-containing plant-based liquid composition and the agent for increasing the β-glucan content of a β-glucan-containing plant-based liquid composition according to the present technique can be set so that the lower limit of the β-glucanase activity per 1 U of α-amylase activity is, for example, 0.0001 U or more, 0.0005 U or more, 0.001 U or more, 0.003 U or more, 0.005 U or more, 0.01 U or more, 0.05 U or more, or 0.1 U or more. The upper limit of β-glucanase activity per 1 U of α-amylase activity can be set to, for example, 10 U or less, 5 U or less, 1 U or less, 0.5 U or less, 0.3 U or less, 0.1 U or less, 0.01 U or less, or 0.005 U or less.

(3) Plant-based materials

As for the plant-based material for which the enzyme preparation for producing β-glucan-containing plant-based liquid composition and the agent for increasing the β-glucan content of a plant-based liquid composition according to the present technique can be used, one or more types of starch-containing plant-based materials that can be used as raw materials for a β-glucan-containing plant-based liquid composition can be freely selected and used, as long as the action and effect of the present technique are not impaired. Specifically, there are no particular limitations on the liquid as long as it contains a plant-based material in water, and it is preferable to use a plant-based material containing starch. Specific examples of starch-containing plant-based materials include (i) a liquid obtained by dispersing a dry powder of a starch-containing plant-based material in water; and (ii) a liquid obtained by crushing and dispersing a starch-containing plant-based material in water, and optionally removing insoluble matter derived from the skin of the plant-based material by any means such as centrifugation, filtration, a filter bag, or a sieve; and a liquid obtained by mixing a dry powder prepared from either liquid (i) or (ii) with water. A preferred example of a starch-containing plant-based material is plant-based milk.

The origin of the plant-based material is not particularly limited as long as it does not impair the actions and effects of the present technique. Examples thereof include beans such as soybeans, peas, lentils, chickpeas, black beans, broad beans, mung beans, lupin beans, and kidney beans; grains such as wheat, barley, oats, sorghum, rice, rye, buckwheat, barnyard millet, foxtail millet, teff, corn, and potato; nuts such as almonds, coconuts, peanuts, cashew nuts, hazelnuts, pecan nuts, macadamia nuts, pistachios, walnuts, Brazil nuts, pili nuts, chestnuts, sesame seeds, and pine nuts; and seeds such as hemp seeds (industrial hemp), chia seeds, chia, amaranth, canary seeds, and flaxseed. In the present technique, among these materials, preferred are cereals, more preferred are wheat, barley, oats, sorghum, rice, rye, buckwheat, barnyard millet, foxtail millet, teff, corn, and potato, and even more preferred are oats.

The starch content of the starch-containing plant-based material is not particularly limited, and a plant-based material containing a preferred amount of starch can be selected depending on the type of β-glucan-containing plant-based liquid composition to be produced, the desired physical properties, and the like. In the present technique, a plant-based material containing, for example, 0.1% by weight or more, 0.5% by weight or more, 1% by weight or more, 2% by weight or more, 3% by weight or more, 4% by weight or more, or 5% by weight or more of starch can be used. In addition, in the present technique, a plant-based material containing, for example, 70% by weight or less, 60% by weight or less, 50% by weight or less, 40% by weight or less, 30% by weight or less, 20% by weight or less, or 10% by weight or less of starch can be used.

The starch-containing plant-based material further contains β-glucan. The amount of β-glucan contained in the starch-containing plant-based material is not particularly limited, and a plant-based material containing a preferred amount of β-glucan can be selected depending on the type of β-glucan-containing plant-based liquid composition to be produced, the desired physical properties, and the like. In the present technique, for example, a plant-based material containing β-glucan at 0.01% by weight or more, 0.05% by weight or more, 0.1% by weight or more, 0.15% by weight or more, or 0.2% by weight or more can be used. In addition, in the present technique, for example, a plant-based material containing β-glucan at 5% by weight or less, 2% by weight or less, 1% by weight or less, 0.8% by weight or less, 0.6% by weight or less, 0.5% by weight or less, or 0.4% by weight or less can be used.

(4) Other components

The enzyme preparation for producing β-glucan-containing plant-based liquid composition and the agent for increasing the β-glucan content of a plant-based liquid composition according to the present technique can be used in combination with other enzymes and other components, as long as the action and effect of the present technique are not impaired. Examples of other enzymes include liquefying enzymes such as β-amylase, maltotriohydrolase, and maltooligosaccharide-forming amylase. Examples of other components that can be used include excipients, pH adjusters, colorants, flavoring agents, disintegrants, lubricants, stabilizers, and other components commonly used in formulations. Furthermore, components with known or future functions can also be used in combination as appropriate depending on the purpose.

2. Method for producing β-glucan-containing plant-based liquid composition and method for increasing the β-glucan content of β-glucan-containing plant-based liquid composition

The method for producing a β-glucan-containing plant-based liquid composition and the method for increasing the β-glucan content of a β-glucan-containing plant-based liquid composition according to the present technique involve an enzyme treating step in which α-amylase and β-glucanase are allowed to treat on a starch-containing plant-based material. Furthermore, if necessary, general steps that are carried out in plant-based liquid composition production methods, such as a recovery step, can also be carried out. Each step will be described in detail below.

(1) Enzyme treating step

The enzyme treating step is a step of allowing α-amylase and β-glucanase to treat on a starch-containing plant-based material. In the enzyme treating step, a step of allowing α-amylase to treat on a starch-containing plant-based material (hereinafter also referred to as the "α-amylase treating step") and a step of allowing β-glucanase to treat on a starch-containing plant-based material (hereinafter also referred to as the "β-glucanase treating step") can be carried out simultaneously or in any order.

(1-1) α-amylase treating step

Various conditions in the α-amylase treating step can be freely set as long as they do not impair the effects of the present technique. For example, the pH, temperature, reaction time, etc. can be set depending on the physicochemical properties of the α-amylase used, such as the optimal pH, stable pH range, optimal temperature, and temperature stability. The pH can be set, for example, to 4 to 10, preferably 5 to 9, more preferably 6 to 8, and even more preferably 6.5 to 7.5. The temperature can be set, for example, to 10°C to 80°C, preferably 20°C to 70°C, more preferably 30°C to 65°C, and even more preferably 40°C to 60°C. The reaction time can be set, for example, to 5 minutes to 48 hours, preferably 30 minutes to 24 hours, and more preferably 1 hour to 5 hours. Optimal reaction conditions can be determined through preliminary experiments.

(1-2) β-glucanase treating step

Various conditions in the β-glucanase treating step can be freely set as long as they do not impair the effects of the present technique. For example, the pH, temperature, reaction time, etc. can be set depending on the physicochemical properties of the β-glucanase used, such as the optimal pH, stable pH range, optimal temperature, and temperature stability. The pH can be set, for example, to 4 to 10, preferably 5 to 9, more preferably 6 to 8, and even more preferably 6.5 to 7.5. The temperature can be set, for example, to 10°C to 80°C, preferably 20°C to 70°C, more preferably 30°C to 65°C, and even more preferably 40°C to 60°C. The reaction time can be set, for example, to 5 minutes to 48 hours, preferably 30 minutes to 24 hours, and more preferably 1 hour to 5 hours. Optimal reaction conditions can be determined through preliminary experiments.

(2) Recovery step

The recovery step is a step of recovering the β-glucan-containing plant-based liquid composition produced through the enzyme treating step. As for the specific recovery method, one or more of the recovery methods generally used in the production of β-glucan-containing plant-based liquid compositions can be freely combined and used depending on the type of β-glucan-containing plant-based liquid composition to be produced.

One embodiment of the method for producing a β-glucan-containing plant-based liquid composition and the method for increasing the β-glucan content of a β-glucan-containing plant-based liquid composition according to the present technique comprises the following steps (1) and (2). An enzyme deactivation step may be added after step (2). The enzymes used in step (2) may be used simultaneously or separately for treatment. When using separately for treatment, the order of treatments is not particularly limited. When using separately for treatment, an enzyme deactivation step may be added between each enzyme treatment, as necessary.

(1) A step of preparing plant-based material
(2) A step of allowing α-amylase and β-glucanase to treat on the prepared plant-based material

One embodiment of the method for producing a β-glucan-containing plant-based liquid composition and the method for increasing the β-glucan content of a β-glucan-containing plant-based liquid composition according to the present technique comprises the following steps (1) to (3). An enzyme deactivation step may be added after step (2). The enzymes used in step (2) may be used simultaneously or separately for treatment. When using separately for treatment, the order of treatments is not particularly limited. When using separately for treatment, an enzyme deactivation step may be added between each enzyme treatment, as necessary.

(1) A step of preparing plant-based material
(2) A step of allowing α-amylase and β-glucanase to treat on the prepared plant-based material
(3) A step of recovering the β-glucan-containing plant-based liquid composition

3. β-glucan-containing plant-based liquid composition

In a β-glucan-containing plant-based liquid composition produced using the enzyme preparation for producing β-glucan-containing plant-based liquid composition according to the present technique or the method for producing a β-glucan-containing plant-based liquid composition according to the present technique, most of the β-glucan is not decomposed, and the β-glucan content is higher than in a plant-based liquid composition obtained by a production method including a step of allowing a general liquefying enzyme to treat. Specific examples of β-glucan-containing plant-based liquid compositions include plant-based milk, beer, vegetable juice, and fruit juice.

EXAMPLES

The present invention will be described in more detail below with reference to examples. Note that the examples described below are representative examples of the present invention and should not be construed as narrowing the scope of the present invention.

1. Raw material

The enzymes and plant-based materials used in the examples are shown in Table 1 below.

[Table 1]

| Name of substance | Product name, content composition, and the like |
|---|---|
| Enzyme preparation 1 | α-amylase, β-glucanase (derived from Bacillus amyloliquefaciens) |
| Enzyme preparation 2 | α-amylase, β-glucanase (derived from Bacillus amyloliquefaciens) |
| Oat flour | Premium oat flour (manufactured by Slow Food) protein content 13.1 wt%, carbohydrate content 67.7 wt%, dietary fiber content 10.1 wt% |

2. Enzyme activity measurement method

[Method for measuring α-amylase activity]

(1) Preparation of substrate solution

Approximately 1 g of potato starch was precisely weighed out and dried at 105°C for 2 hours, and its weight loss was measured. Potato starch equivalent to 1.000 g of the dried product was accurately weighed and placed in a beaker. 20 mL of water was added, and while mixing well, 5 mL of sodium hydroxide solution (2 → 25) was gradually added to form a paste. Next, the mixture was heated in a water bath with stirring for 3 minutes, after which 25 mL of water was added. After cooling, the mixture was accurately neutralized with 2 mol/L hydrochloric acid test solution, 10 mL of 1 mol/L acetic acid/sodium acetate buffer (pH 5.0) was added, and water was added to make the total volume exactly 100 mL.

(2) Measurement of absorbance

10 mL of the substrate solution prepared above was accurately measured and heated at $37\pm0.5$°C for 10 minutes. Then, 1 mL of the sample solution was accurately added and immediately shaken. After leaving this solution at $37\pm0.5$°C for accurately 10 minutes, 1 mL of this solution was accurately measured and added to 10 mL of 0.1 mol/L hydrochloric acid test solution, followed by immediate shaking. Next, 0.5 mL of this solution was accurately measured and 10 mL of 0.0002 mol/L iodine test solution (Japanese Pharmacopoeia) was accurately added. After shaking, the absorbance (AT) at a wavelength of 660 nm was measured, using water as a control. The 0.0002 mol/L iodine test solution (Japanese Pharmacopoeia) was prepared by adding 10 mL of water to 12.7 g of iodine and 25 g of potassium iodide, mixing thoroughly, adding water to make 100 mL, and then diluting 2500-fold with water. Separately, 1 mL of water was accurately added in place of the sample solution, and the same operation was performed to measure the absorbance (AB).

(3) Activity calculation method

Under these conditions, the amount of enzyme that reduces the color of potato starch caused by iodine by 10% in 1 minute was defined as 1 unit, and the activity was calculated using the following formula.

$$\alpha\text{-amylase activity (U/g, U/mL)} = \{(AB-AT)/AB\}\times 1/W$$

AT: absorbance of sample solution
AB: absorbance of blank solution
W: amount of sample in 1 mL of sample solution (g or mL)

[Method for measuring β-glucanase activity]

(1) Preparation of substrate solution

Sodium carboxymethylcellulose powder manufactured by Fujifilm Wako Pure Chemical Corporation was suspended in 50 mM phosphate buffer, pH 7.0, to a final concentration of 0.55% to prepare a substrate solution.

(2) Measurement of absorbance

1.8 mL of the substrate solution (pH 7.0) prepared above was added to a test tube and shaken. The tube was stoppered with a marble and left at $52 \pm 0.5$°C for 10 minutes. 0.2 mL of sample solution was then added and immediately shaken. This solution was then left at $52 \pm 0.5$°C for exactly 30 minutes, after which 3 mL of 0.5% dinitrosalicylic acid test solution was added and shaken. The tube was then stoppered with a marble and heated in a boiling water bath for exactly 20 minutes, then immediately cooled under running water. After cooling, the absorbance (AT) at a wavelength of 540 nm was measured using water as a control. Separately, 1.8 mL of substrate solution (pH 7.0) was measured, shaken, and left at $52 \pm 0.5$°C for exactly 30 minutes. 3 mL of 0.5% dinitrosalicylic acid test solution was then added and shaken. 0.2 mL of sample solution was added to this solution, shaken, and the same operation was performed to measure the absorbance (AB).

(3) Preparation of glucose calibration curve

0.06 g of D(-)-glucose was weighed out and dissolved in water to make 100 mL (4 μmol/mL). 2.5 mL, 5 mL, and 10 mL of this solution were measured and water was added to make 20 mL (0.5 μmol/mL, 1 μmol/mL, and 2 μmol/mL). 2 mL each of water and 0.5 μmol/mL, 1 μmol/mL, and 2 μmol/mL glucose solutions were placed in test tubes, and 3 mL of 0.5% dinitrosalicylic acid was added. The tubes were shaken and stoppered with marbles. The tubes were heated in a boiling water bath for exactly 20 minutes and then immediately cooled under running water. After cooling, the absorbance at 540 nm was measured using water as a control.

(4) Activity calculation method

Under these conditions, the amount of enzyme that produces 1 μmol of glucose in 17 hours was defined as 1 unit, and the enzyme activity was calculated using the following formula.

$$\beta\text{-glucanase activity (U/g, U/mL)} = ((AT-AB)-b)/a/0.2 \times n$$

AT: absorbance of sample solution
AB: absorbance of blank solution
a: slope of the calibration curve obtained with glucose solution
b: y-intercept of the calibration curve obtained with glucose solution
0.2: amount of enzyme
n: dilution factor per 1 mL of sample

3. Experimental Examples

<Experimental Example 1>

In Experimental Example 1, the influence of difference in the amount of enzyme used on β-glucan in a β-glucan-containing plant-based liquid composition was investigated. In Experimental Example 1, oat flour (powder) was used as an example of a plant-based material to produce plant-based milk (oat milk) as an example of a β-glucan-containing plant-based liquid composition.

(1) Production of plant-based milk and calculation of β-glucan decomposition rate (%)

18.1 g of oat flour was mixed with 150 g of water and suspended in a 300 mL Erlenmeyer flask. The enzyme preparation listed in Table 2 below was added to this suspension and allowed to react at 60°C for 0.5 hours. After the reaction, the mixture was heated at 95°C for 5 minutes to inactivate the enzyme, resulting in an enzyme-treated sample. A similar treatment was performed without the addition of amylase, resulting in an enzyme-untreated sample. β-glucan in the enzyme-treated sample was quantified using a β-glucan quantification kit (Megazyme). The β-glucan decomposition rate (%) was calculated using the following formula from the amount of β-glucan reduced relative to the amount of β-glucan in the enzyme-untreated sample.

β-glucan decomposition rate = {(β-glucan amount in enzyme-untreated sample-β-glucan amount in enzyme-treated sample)/β-glucan amount in enzyme-untreated sample} × 100

(2) Results

The results are shown in Table 2.

[Table 2]

|  | Sample | | | | | | |
|---|---|---|---|---|---|---|---|
|  | 1 | 2 | 3 | 4 | 5 | 6 | 7 |
| Addition amount (%) of enzyme preparation 1 | 0.1 | 0.05 | 0.01 | 0.005 | - | - | - |
| Addition amountt (%) of enzyme preparation 2 | - | - | - | - | 0.1 | 0.01 | 0.001 |
| α-amylase addition activity (U/mL) | 5.5 | 2.7 | 0.55 | 0.27 | 0.76 | 0.08 | 0.01 |

(continued)

| | Sample | | | | | | |
|---|---|---|---|---|---|---|---|
| | 1 | 2 | 3 | 4 | 5 | 6 | 7 |
| β-glucanase addition activity (U/mL) | 1.3 | 0.66 | 0.13 | 0.066 | 0.003 | 0.0003 | 0.00003 |
| β-glucanase addition activity (U) per 1 g of β-glucan in the raw material | 650 | 330 | 65 | 33 | 1.5 | 0.15 | 0.015 |
| β-glucan decomposition rate (%) | 69.5 | 52.6 | 5.4 | 1.3 | 5 | 3.6 | 0 |

(3) Discussion

In the production of a β-glucan-containing plant-based liquid composition, it was thought that in order to prevent the decomposition of β-glucan, it was necessary to completely remove β-glucanase from the enzyme preparation. However, unexpectedly, as shown in Table 2, it was found that β-glucan remained even when an enzyme preparation containing β-glucanase was used.

In particular, it was confirmed that by adding 300 U or less of β-glucanase per 1 g of β-glucan in the raw material, 50% or more of the β-glucan can be retained.

<Experimental Example 2>

In Experimental Example 2, the influence of extending the reaction time of the enzyme preparation on the β-glucan in the β-glucan-containing plant-based liquid composition was investigated. In Experimental Example 2, similar to Experimental Example 1, oat flour (powder) was used as an example of the plant-based material to produce plant-based milk (oat milk) as an example of the β-glucan-containing plant-based liquid composition.

(1) Production of plant-based milk and calculation of β-glucan decomposition rate (%)

Plant-based milk was produced in the same manner as in Experimental Example 1, except that the amount of enzyme preparation and reaction time were as shown in Table 3 below, and the β-glucan decomposition rate (%) was calculated.

(2) Results

The results are shown in Table 3.

[Table 3]

| | Sample |
|---|---|
| | 8 |
| Addition amount (%) of enzyme preparation 2 | 0.5 |
| α-amylase addition activity (U/mL) | 4 |
| β-glucanase addition activity (U/mL) | 0.015 |
| β-glucanase addition adtivity (U) per 1 g of β-glucan in the raw material | 7.5 |
| Reaction time (hr) | β-glucan decomposition rate (%) |
| 1 | 3.6 |
| 2 | 1.1 |
| 4 | 5 |
| 8 | 3.9 |
| 16 | 3 |

(3) Discussion

As shown in Table 3, no significant change in the decomposition rate of β-glucan was observed even when the reaction time was extended. This result indicates that the decomposition reaction of β-glucan does not proceed even when the reaction time is extended.

**Claims**

1. An enzyme preparation for producing β-glucan-containing plant-based liquid composition, which comprises α-amylase and β-glucanase.

2. The enzyme preparation for producing β-glucan-containing plant-based liquid composition, according to Claim 1, wherein
the content of the β-glucanase per 1 g of β-glucan in the raw materials of the β-glucan-containing plant-based liquid composition is 0.001 U or more and 300 U or less.

3. The enzyme preparation for producing β-glucan-containing plant-based liquid composition according to Claim 2, wherein
the plant-based liquid composition is plant-based milk.

4. An agent for increasing the β-glucan content of a plant-based liquid composition, which comprises α-amylase and β-glucanase.

5. A method for producing a β-glucan-containing plant-based liquid composition, comprising an enzyme treating step of allowing α-amylase and β-glucanase to treat on a starch-containing plant-based material.

6. The method for producing a β-glucan-containing plant-based liquid composition according to Claim 5, wherein
the enzyme treating step comprises treating the β-glucanase at 0.001 U or more and 300 U or less per 1 g of β-glucan in the starch-containing plant-based material.

## INTERNATIONAL SEARCH REPORT

International application No.

**PCT/JP2024/007761**

**A. CLASSIFICATION OF SUBJECT MATTER**

*C12N 9/26*(2006.01)i; *A23L 33/21*(2016.01)i; *C12N 9/28*(2006.01)i
FI:   C12N9/26 A; C12N9/28; A23L33/21

According to International Patent Classification (IPC) or to both national classification and IPC

**B. FIELDS SEARCHED**

Minimum documentation searched (classification system followed by classification symbols)

C12N9/26; A23L33/21; C12N9/28

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | EP 4005398 A2 (POLAR GLUCAN OY) 01 June 2022 (2022-06-01)<br>claims, paragraph [0039], example 1 | 1-6 |
| X | US 2022/0240547 A1 (JASPER PRODUCTS, L.L.C.) 04 August 2022 (2022-08-04)<br>claims, examples 1-7 | 1-6 |
| P, X | JP 2023-64843 A (GUN EI CHEMICAL INDUSTRY CO., LTD.) 12 May 2023 (2023-05-12)<br>claims, paragraphs [0075], [0087] | 1-6 |
| A | WO 2022/244875 A1 (AMANO ENZYME INC.) 24 November 2022 (2022-11-24)<br>claims, paragraph [0073], examples | 1-6 |
| A | JP 2020-519245 A (THE QUAKER OATS COMPANY) 02 July 2020 (2020-07-02)<br>claims | 1-6 |
| A | JP 2016-506732 A (OATLY AB) 07 March 2016 (2016-03-07)<br>claims, paragraphs [0024], [0027], examples | 1-6 |

☑ Further documents are listed in the continuation of Box C.       ☑ See patent family annex.

| | | |
|---|---|---|
| \* | Special categories of cited documents: | |
| "A" | document defining the general state of the art which is not considered to be of particular relevance | |
| "D" | document cited by the applicant in the international application | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **25 March 2024** | **09 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

EP 4 674 956 A1

**INTERNATIONAL SEARCH REPORT**

| International application No. |
|---|
| **PCT/JP2024/007761** |

**C. DOCUMENTS CONSIDERED TO BE RELEVANT**

| Category* | Citation of document, with indication, where appropriate, of the relevant passages | Relevant to claim No. |
|---|---|---|
| A | JP 2002-527089 A (CEREAL BASE CEBA AB) 27 August 2002 (2002-08-27) claims, examples | 1-6 |

Form PCT/ISA/210 (second sheet) (July 2022)

13

## INTERNATIONAL SEARCH REPORT
### Information on patent family members

International application No.

**PCT/JP2024/007761**

| Patent document cited in search report | Publication date (day/month/year) | Patent family member(s) | Publication date (day/month/year) |
|---|---|---|---|
| EP 4005398 A2 | 01 June 2022 | (Family: none) | |
| US 2022/0240547 A1 | 04 August 2022 | (Family: none) | |
| JP 2023-64843 A | 12 May 2023 | (Family: none) | |
| WO 2022/244875 A1 | 24 November 2022 | CA 3219725 A1<br>claims, examples | |
| JP 2020-519245 A | 02 July 2020 | US 2018/0327792 A1<br>claims<br>WO 2018/226358 A1<br>EP 3621460 A1<br>KR 10-2020-0007009 A<br>CN 110621167 A | |
| JP 2016-506732 A | 07 March 2016 | US 2015/0351432 A1<br>claims, paragraphs [0020], [0023], examples<br>WO 2014/123466 A1<br>EP 2953482 A1<br>KR 10-2015-0113200 A<br>CN 105007757 A | |
| JP 2002-527089 A | 27 August 2002 | US 2002/0081367 A1<br>claims, examples<br>WO 2002/065855 A2<br>EP 1123012 A2<br>CN 1323167 A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9505204 W